# FASCICULE DE BREVET EUROPEEN

(11) **EP 1 355 625 B1**
(45) Date de publication et mention de la délivrance du brevet: **15.11.2006**
(21) Numéro de dépôt: 02700319.3
(22) Date de dépôt: 14.01.2002
(51) Int. Cl.: A61K 8/81, A61Q 1/02, A61Q 19/00

(54) **COMPOSITIONS A EFFET OPTIQUE, NOTAMMENT COSMETIQUES**
OPTISCH WIRKSAME ZUSAMMENZETUNGEN, INSBESONDERE FÜR DIE KOSMETIK
COMPOSITIONS, IN PARTICULAR COSMETIC, WITH OPTICAL PROPERTY

(30) Priorité: 15.01.2001 FR 0100481
(43) Date de publication de la demande: 29.10.2003
(73) Titulaire: L'ORÉAL, 75008 Paris (FR)
(72) Inventeur: MAMANE, Maurice, F-94600 CHOISY LE ROI (FR)
(74) Mandataire: Poulin, Gérard
(86) Numéro de dépôt international: PCT/FR2002/000122
(87) Numéro de publication internationale: WO 2002/055051

(56) Documents cités:
- EP-A- 0 754 446
- EP-A- 0 858 795
- EP-A- 0 998 905
- WO-A-00/35961
- HOURDET D ET AL: "Synthesis of thermoassociative copolymers" POLYMER, ELSEVIER SCIENCE PUBLISHERS B.V, GB, vol. 38, no. 10, 1 mai 1997 (1997-05-01), pages 2535-2547, XP004059755 ISSN: 0032-3861

## Description

La présente invention a trait à des compositions à effet optique. L'invention a également trait à l'utilisation de polymères spécifiques en particulier pour améliorer la tenue de dépôts ou films obtenus à partir de ces compositions à effet optique.

Les compositions à effet optique selon l'invention peuvent se présenter sous la forme d'émulsions ou de dispersions et sont essentiellement des compositions pour une application topique et notamment cosmétique.

Le domaine technique de l'invention peut être défini de manière générale comme celui des compositions à effet optique.

Les compositions à effet optique sont, par exemple, dans le domaine cosmétique, des produits de maquillage ou de soin apportant, après application une modification de l'aspect du visage ou du corps appréciable de façon visuelle. Citons parmi ces compositions les fonds de teint, les mascaras et les produits apportant de la couvrance ou du "Soft Focus".

Les propriétés optiques (aspect visuel, couvrance, couleur, matité) de ces compositions après application sur la peau ou sur des fibres kératiniques diminuent avec le temps, et particulièrement dans des conditions climatiques chaudes et humides. La modification du dépôt ou film cosmétique induit par l'humidité et la chaleur peut se traduire par une perte des propriétés de non-transfert, et/ou des changements visuels indésirables liés à la mauvaise tenue de ce dépôt.

En outre, ces produits ou compositions ou formules à effet optique, qui sont, à température ambiante, généralement sous forme fluide ou gélifiée, présentent de nombreuses limites quant à leur utilisation. En effet :
- les formules fluides sont difficiles à appliquer en raison de leur faible viscosité qui provoquent des coulures lors de leur application ;
- les formules gélifiées sont obtenues à l'aide de gélifiants hydrophiles, tels que les acides polyacryliques réticulés, en particulier les dérivés commercialisés sous le nom de CARBOPOL^{®}, qui limitent les formes galéniques que l'on peut obtenir.

En d'autres termes, si l'on souhaite à l'heure actuelle disposer d'un gel agréable à appliquer sur la peau, on doit obligatoirement faire appel à une composition qui est, elle-même, gélifiée avec tous les inconvénients associés. La variété des textures, que peut revêtir une composition à effet optique susceptible de donner un gel lors de son application sur la peau, est donc extrêmement limitée.

Il existe donc un besoin pour une composition à effet optique qui donne - après application - des dépôts ou films présentant une excellente tenue même dans des conditions d'atmosphère humide et/ou chaude. Ladite composition pouvant revêtir une large gamme de textures, notamment fluides ou gélifiées, en particulier à température ambiante, permettant une application aisée.

Le but de la présente invention est, entre autres, de répondre à ce besoin.

Ce but et d'autres encore sont atteints, conformément à l'invention, par la composition cosmétique de la revendication 1.

De manière surprenante, il a été montré que les compositions cosmétiques à effet optique, selon l'invention, qui comprennent le polymère spécifique, tel qu'il est défini ci-dessus, permettent de répondre aux besoins énumérés ci-dessus et de répondre à l'ensemble des exigences mentionnées dans ce qui précède.

En substance, les propriétés d'un dépôt ou film obtenu à partir de la composition à effet optique selon l'invention incluant le polymère spécifique défini ci-dessus, restent constantes, ne diminuent pas, ne se dégradent pas avec le temps, et ce même lorsque le dépôt ou le film est en contact avec une atmosphère humide et/ou chaude. En outre, la présence de ces polymères dans la composition ne limite pas le type de texture de la composition qui peut être par exemple fluide ou encore gélifiée à la température ambiante.

Les films ou dépôts obtenus à partir des compositions selon l'invention, ne perdent absolument pas, au contraire des compositions de l'art antérieur qui contiennent par exemple, des gélifiants de type polyacrylique, leurs propriétés de non transfert avec le temps et ce même avec une exposition à une atmosphère humide et/ou chaude.

En d'autres termes, les dépôts obtenus après application ont une faible sensibilité à l'humidité, les films ou dépôts obtenus à partir des compositions de l'invention conservent une excellente tenue quelle que soit l'humidité relative et/ou la température.

La tenue de ces dépôts et/ou films est évaluée par exemple à partir de leur pouvoir collant superficiel ou "Tack" dont la diminution est un facteur favorable à l'égard des propriétés de non transfert et/ou de tenue. Des mesures ont montré que le pouvoir collant superficiel des films ou dépôts obtenus à partir des compositions à effet optique de l'invention reste nul, sur une longue durée, quelles que soient l'humidité relative et la température auxquelles ils se trouvent exposés et que dans tous les cas, au pire, le pouvoir collant superficiel des films ou dépôts obtenus à partir des compositions de l'invention reste inférieur à 0,2 J/m².

Les compositions selon l'invention peuvent se présenter sous toute forme à une température qui est inférieure à leur température de gélification, par exemple, à la température ambiante.

C'est-à-dire qu'à une température inférieure à leur température de gélification, par exemple, à la température ambiante, les compositions de l'invention peuvent avoir une viscosité faible ou bien élevée. Cela signifie que « dans le pot », c'est-à-dire préalablement à une augmentation de température intervenant généralement au moment de leur utilisation, par exemple de leur application, généralement sur la peau, (température voisine de 32°C), la gamme des textures accessibles par les compositions de l'invention n'est pas limitée.

Au contraire, les compositions analogues de l'art antérieur ne peuvent revêtir qu'un nombre extrêmement limité de textures et doivent obligatoirement se présenter sous forme gélifiée, si l'on souhaite obtenir un gel, par exemple, lors de l'application.

Selon l'invention, il n'y a aucune restriction sur la forme de la composition à température ambiante.

On peut faire varier à volonté la forme de la composition, et cependant, quelle que soit cette forme, par exemple fluide ou gélifiée, on aura, lors de l'application, par exemple sur la peau (dont la température est généralement voisine de 32°C) qui s'accompagne d'une augmentation de température et/ou d'une évaporation des espèces volatiles de la composition, une apparition du pouvoir gélifiant du polymère et gélification.

Cette texture de gel est d'une application aisée et agréable.

Le pouvoir gélifiant des polymères selon l'invention peut donc apparaître lors de l'application du fait d'une augmentation de température, et/ou de l'évaporation des espèces volatiles de la composition conduisant à une augmentation de la concentration.

En d'autres termes, les compositions à effet optique selon l'invention sont, par exemple, sous forme fluide à la température ambiante, et leur viscosité devient importante à une température plus élevée, qui est celle, par exemple, rencontrée lors de l'application sur la peau. Une telle gélification, provoquée à la température de la peau, généralement voisine de 32°C, permet une application aisée des compositions ou formules selon l'invention.

L'invention concerne, en outre, l'utilisation du polymère, tel que décrit dans la présente description, pour annuler ou diminuer le pouvoir collant superficiel d'un film ou dépôt obtenu à partir d'une composition à effet optique contenant ledit polymère.

L'invention a trait enfin à l'utilisation du polymère, tel que décrit dans la présente description, pour maintenir (au cours du temps) la tenue d'un film ou dépôt obtenu à partir d'une composition à effet optique contenant ledit polymère.

De telles utilisations de ces polymères pour améliorer les propriétés du film ou dépôt obtenu à partir d'une composition à effet optique contenant ce polymère, ne sont ni décrites, ni suggérées dans l'art antérieur. En particulier, ces films ou dépôts ont un pouvoir collant superficiel nul ou inférieur à 0,2 J/m² sur une longue durée (par exemple de 1 à 10 heures) même lorsqu'ils sont exposés à une atmosphère chaude et/ou humide à savoir généralement à une température de 25° à 45°C et/ou à une humidité relative (HR) de 40 à 95%. L'influence exercée par les polymères décrits dans la demande lorsqu'ils sont inclus dans une composition à effet optique sur le "Tack", la tenue et le non-transfert n'a jamais été décrite ni évoquée dans l'art antérieur.

Le constituant essentiel des compositions selon l'invention est un polymère comprenant des unités hydrosolubles et dés unités présentant dans l'eau une température inférieure critique de démixtion (LCST), encore appelées « unités à LCST ».

A ce propos, il est' utile de rappeler que par unités à LCST, on entend des unités dont la solubilité dans l'eau est modifiée au-delà d'une certaine température. Il s'agit d'unités présentant une température de démixtion par chauffage (ou point de trouble) définissant leur zone de solubilité dans l'eau. La température de démixtion minimale obtenue en fonction de la concentration en polymère constitué uniquement d'unités à LCST est appelée « LCST » (Lower Critical Solution Température). Pour chaque concentration en polymère à LCST, une température de démixtion par chauffage est observée. Elle est supérieure à la LCST qui est le point minimum de la courbe. En dessous de cette température, le polymère est soluble dans l'eau, au-dessus de cette température, le polymère perd sa solubilité dans l'eau.

Ces unités à LCST du polymère utilisé dans la composition à effet optique selon l'invention répondent à une définition spécifique qui, de manière fondamentale, permet de communiquer au polymère toutes les propriétés avantageuses, décrites plus haut relatives notamment à la suppression du pouvoir collant superficiel, à l'amélioration de la tenue et des propriétés de non-transfert, de films ou dépôts obtenus à partir de ces compositions.

Les unités à LCST du polymère ont, selon l'invention, une température de démixtion par chauffage de 5 à 40°C pour une concentration massique dans l'eau de 1 % à 25 % en poids desdites unités à LCST.

De préférence, la température de démixtion par chauffage en solution aqueuse des unités à LCST du polymère est de 10 à 35°C pour une concentration massique dans l'eau de 1 % à 25 % desdites unités à LCST.

Le polymère ayant la structure décrite plus haut avec des unités hydrosolubles et des unités à LCST spécifiques définies ci-dessus présente en solution aqueuse, des propriétés de gélification au-delà d'une température critique, ou propriétés de thermogélification.

Ces propriétés de gélification par chauffage observées au-delà de la température de démixtion des chaînes à LCST sont décrites dans l'art antérieur, notamment dans les documents [1], [2] et [3]. Elles sont dues à l'association des chaînes à LCST au sein de microdomaines hydrophobes au-delà de leur température de démixtion, formant ainsi des noeuds de réticulation entre les chaînes principales.

Ces propriétés gélifiantes sont observées lorsque la concentration en polymère est suffisante pour permettre des interactions entre Les unités à LCST portées par des macromolécules différentes. La concentration minimale nécessaire, appelée « concentration critique d'agrégation ou CAC », est évaluée par des mesures de rhéologie : il s'agit de la concentration à partir de laquelle la viscosité d'une solution aqueuse des polymères de l'invention devient supérieure à la viscosité d'une solution du polymère équivalent ne comportant pas de chaînes à LCST.

Au-delà de la CAC, les polymères de l'invention présente des propriétés de gélification lorsque la température devient supérieure à une valeur critique appelée « température de gélification ou Tgel ». D'après les données de la littérature, un bon accord existe entre Tgel et la température de démixtion des chaînes à LCST, dans les mêmes conditions de concentrations. La température de gélification d'une solution aqueuse d'un polymère de l'invention est déterminée par des mesures de rhéologie : il s'agit de la température à partir de laquelle la viscosité de la solution d'un polymère de l'invention devient supérieure à la viscosité d'une solution du polymère équivalent ne comportant pas de chaînes à LCST.

Les polymères de l'invention se caractérisent par une température de gélification spécifique généralement de 5 à 40°C, de préférence de 10 à 35°C, pour une concentration massique dans l'eau, de 2 % à 50% en poids.

Il est à noter que cette concentration n'est pas, généralement, celle des compositions de l'invention, mais qu'elle peut être atteinte du fait de l'évaporation des espèces volatiles de la composition, par exemple lors de l'application, provoquant ainsi, l'apparition du pouvoir gélifiant du polymère.

En fonction de cette température de gélification spécifique dans les conditions de l'utilisation, par exemple lors de l'application sur la peau, les compositions de l'invention présentent une viscosité faible ou élevée et donc une grande variété de formes à température ambiante, et leurs effets favorables sur le dépôt ou film obtenu, lors de leur application, par exemple sur la peau, sont toujours conservés.

Des polymères comportant, à l'instar de ceux mis en oeuvre dans les compositions de l'invention, des unités hydrosolubles et des unités à LCST et possédant des propriétés de gélification par chauffage observées au-delà de la température de démixtion des chaînes à LCST sont décrites dans les documents déjà cités plus haut.

Le document [1] est relatif au thermo-épaississement réversible de solutions aqueuses de copolymère comprenant un squelette hydrosoluble d'acide polyacrylique avec des greffons de poly(oxyde d'éthylène) (POE ou PEO en anglais).

Le document [2] concerne le comportement thermo-épaississant en solution aqueuse de polymères comportant un squelette d'acide 2-acrylamido-2-méthylpropanesulfonique (AMPS) et des chaînes latérales de poly(oxyde éthylène).

De même, le document [3] décrit la thermo-association réversible de copolymères à squelette hydrosoluble polyacrylique ou à base de AMPS avec des greffons de POE.

Les polymères, tels que ceux mentionnés dans les documents [1], [2] et [3] trouvent, en particulier, leur application dans l'industrie pétrolière.

Ainsi, le document [4] décrit-il des polymères thermoviscosifiants à squelette hydrosoluble comportant des segments, ou portant des chaînes latérales, à LCST qui peuvent être utilisés notamment en tant qu'agents épaississants, constituants de fluides de forage ou autres fluides, et fluides de nettoyage industriel.

Le document [5] décrit des polymères analogues à ceux du document [4] et leur utilisation en tant qu'agent anti-sédimentation de suspensions, éventuellement dans les préparations cosmétiques.

Il est à noter qu'aucun des documents [1] à [5] ne mentionne l'incorporation des polymères dans des compositions à effet optique.

Le document [6] décrit également les copolymères comprenant un squelette constitué par des unités sensibles au pH, par exemple des unités polyacryliques, et des unités sensibles à la température, greffées sur ce squelette. Ces copolymères présentent des propriétés de gélification en température et ils sont utilisés pour la libération et le relargage contrôlé de principes actifs ou pharmaceutiques et, éventuellement cosmétiques, par application topique.

Les unités sensibles à la température des copolymères de ce document ne sont pas les unités à LCST spécifiques des polymères de l'invention. De plus, les polymères selon le document [6] se caractérisent par l'opacité extrêmement gênante des gels obtenus en température, ce qui n'est pas le cas des polymères mis en oeuvre dans les compositions à effet optique de l'invention.

En fait, le polymère de ce document est fondamentalement différent de celui de l'invention car il présente globalement pour l'ensemble du polymère une LCST dans la plage de températures de 20 à 40°C, et non une température de gélification.

Les documents [7] et [8] décrivent des systèmes polymères à gélification réversible, comprenant un composant sensible capable d'agrégation, en réponse à un changement dans un « stimulus » extérieur, et un composant structural. Le stimulus extérieur peut être, par exemple, la température.

Le composant sensible au stimulus extérieur est fondamentalement différent des unités à LCST de la présente demande. En effet, ces composants sensibles au stimulus extérieur sont en fait constitués par au moins un fragment hydrophile et un fragment hydrophobe. Ainsi, le composant sensible peut être un copolymère bloc, tel qu'un « poloxamer », par exemple un PLURONIC^{®}, qui est un polymère bloc d'oxyde éthylène (soluble) et d'oxyde de propylène (insoluble), qui s'agrège au niveau microscopique au-delà d'une température critique ne correspondant pas à une LCST.

Le document [7] concerne plus particulièrement un réseau polymérique formé d'un squelette hydrosoluble polyacrylique et d'un composant sensible de PLURONIC^{®}. qui est enchevêtré dans ledit squelette, sans liaison covalente, ce réseau a donc une structure particulière qui n'a rien de commun avec le polymère mis en oeuvre dans l'invention. Par contre, dans le document [8], il est question de polymères avec des liaisons covalentes.

Ces polymères ont des propriétés de gélification par chauffage et ils peuvent être utilisés dans le domaine pharmaceutique pour la délivrance de médicaments et dans de nombreux autres domaines y compris le domaine des cosmétiques. En ce qui concerne les applications topiques et cosmétiques citées, seuls des exemples d'émulsions telles que des produits solaires et de soin, mais non des compositions à effet optique sont mentionnés. Elles contiennent toutes un tensioactif neutre, anionique, ou cationique, ainsi que le polymère jouant le rôle de gélifiant.

L'utilisation des polymères dans des compositions particulières à effet optique n'est jamais évoquée, ni leurs propriétés à l'état de film, et encore moins leur effet favorable entre autres, sur la tenue, et le pouvoir collant superficiel de ces films. Il en est de même pour les effets de stabilisation et de texture obtenus dans l'invention.

Dans ces formulations, le composant sensible du système polymère a un comportement différent de celui d'unités à LCST, telles que celles du polymère de l'invention, lors du chauffage. Ainsi, lorsqu'on chauffe ledit composant sensible (par exemple, poloxamer), à environ 30-40°C, il présente une température de micellisation, c'est-à-dire une agrégation à l'échelle microscopique, puis lorsqu'on le chauffe davantage, une température de LCST très supérieure. Cette LCST correspond à une agrégation à l'échelle macroscopique entre les chaînes à LCST. Cette différence de comportement entre les polymères à unités LCST de notre invention et les systèmes' polymères utilisant des poloxamers est expliquée dans WO-A-97 00275 [8] aux pages 16 et 17, où il est dit que la gélification et la LCST sont observées à des températures qui diffèrent d'environ 70°C, la température de gélification correspondant à la température de micellisation du composant sensible. En outre, il n'est pas possible, du fait de la synthèse utilisée dans le document [8], de contrôler parfaitement la structure et les propriétés du polymère final obtenu, comme c'est le cas dans les compositions de l'invention.

On connaît encore par le document [9] des compositions cosmétiques utilisant un système polymère à thermogélification réversible, comprenant l'acide polyacrylique et un poloxamer comme dans les documents [7] et [8]. De nouveau, le système polymère de ces documents est fondamentalement différent de ceux mis en oeuvre dans les compositions de l'invention, car il s'aggrège au niveau microscopique au-delà d'une température critique ne correspondant pas à une LCST.

WO-A-00 35961 [10] décrit la préparation de polymères ayant des propriétés d'épaississement en température par polymérisation en émulsion et l'utilisation de ces polymères dans des compositions pharmaceutiques et cosmétiques. Ces polymères peuvent être des copolymères ayant des unités hydrosolubles et des unités à LCST à base d'oxyde d'alkylène. Il est prévu d'ajouter des tensioactifs non ioniques aux polymères pour renforcer leurs propriétés thermo-épaississantes.

Il ressort de ce qui précède que la mise en oeuvre dans des compositions à effet optique des polymères selon l'invention, présentant des unités à LCST spécifiques n'est jamais décrite ni suggérée dans l'art antérieur.

A fortiori, ne sont pas décrites les propriétés à l'état de films de telles compositions et tous les effets et avantages spécifiques sur les propriétés de ces films liées à la mise en oeuvre des polymères spécifiques décrits dans l'invention.

Les polymères utilisés dans l'invention peuvent être des, polymères séquencés (ou blocs), ou des polymères greffés, qui comprennent, d'une part, des unités hydrosolubles et, d'autre part, des unités à LCST telles que définies ci-dessus.

On précise que, dans le présent texte, les unités hydrosolubles ou les unités à LCST des polymères mis en oeuvre selon l'invention sont définies comme n'incluant pas les groupes liant entre elles, d'une part, lesdites unités hydrosolubles et, d'autre part, lesdites unités à LCST.

Lesdits groupes de liaison sont issus de la réaction, lors de la préparation du polymère, des sites réactifs portés, d'une part, par les précurseurs desdites unités hydrosolubles et, d'autre part, par les précurseurs desdites unités à LCST.

Les polymères employés dans le cadre de l'invention peuvent donc être des polymères séquencés, comprenant par exemple des séquences constituées d'unités hydrosolubles alternées avec des séquences à LCST. '

Ces polymères peuvent également se présenter sous la forme de polymères greffés dont le squelette est formé d'unités hydrosolubles, ledit squelette étant porteur de greffons constitués d'unités à LCST.

Lesdits polymères peuvent être partiellement réticulés.

Par unités hydrosolubles, on entend généralement que ces unités sont des unités solubles dans l'eau, à une température de 5 à 80°C, à raison d'au moins 10 g/l, de préférence d'au moins 20 g/l.

Toutefois, on entend également par unités hydrosolubles des unités ne possédant pas obligatoirement la solubilité ci-dessus mentionnée, mais qui en solution aqueuse à 1 % en poids, de 5 à 80°C, permettent l'obtention d'une solution macroscopiquement homogène et transparente, c'est-à-dire ayant une valeur de transmittance maximum de la lumière, quelle que soit la longueur d'onde comprise entre 400 et 800 nm, à travers un échantillon de 1 cm d'épaisseur, d'au moins 85 %, de préférence d'au moins 90 %.

Ces unités hydrosolubles ne présentent pas de température de démixtion par chauffage de type LCST.

Ces unités hydrosolubles sont en totalité ou en partie susceptibles d'être obtenues par polymérisation, notamment radicalaire, ou par polycondensation, ou encore sont constituées en totalité ou en partie par des polymères naturels ou naturels modifiés, existants.

A titre d'exemple, les unités hydrosolubles ou sont en totalité ou en partie susceptibles d'être obtenus par polymérisation, notamment radicalaire, d'au moins un monomère choisi parmi les monomères suivants :
- l'acide (méth)acrylique ;
- les monomères vinyliques de formule (I) suivante : 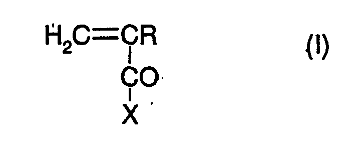
dans laquelle :
- R est choisi parmi H, -CH₃, -C₂H₅ ou -C₃H₇ ; et
- X est choisi parmi :
   - les oxydes d'alkyle de type -OR' où R' est un radical hydrocarboné, linéaire ou ramifié, saturé ou insaturé, ayant de 1 à 6 atomes de carbone, éventuellement substitué par au moins un atome d'halogène (iode, brome, chlore, fluor) ; un groupement sulfonique (-SO₃⁻), sulfate (-SO₄⁻), phosphate (-PO₄H₂) ; hydroxy (-OH) ; amine primaire (-NH₂) ; amine secondaire (-NHR₁), tertiaire (-NR₁R₂) ou quaternaire (-N⁺R₁R₂R₃) avec R₁, R₂ et R₃ étant, indépendamment l'un de l'autre, un radical hydrocarboné, linéaire ou ramifié, saturé ou insaturé ayant 1 à 6 atomes de carbone, sous réserve que la somme des atomes de carbone de R' + R₁ + R₂ + R₃ ne dépasse pas 7 ; et
   - les groupements -NH₂, -NHR₄ et -NR₄R₅ dans lesquels R₄ et R₅ sont indépendamment l'un de l'autre des radicaux hydrocarbonés, linéaires ou ramifiés, saturés ou insaturés ayant 1 à 6 atomes de carbone, sous réserve que le nombre total d'atomes de carbone de R₄ + R₅ ne dépasse pas 7, lesdits R₄ et R₅ étant éventuellement substitués par un atome d'halogène (iode, brome, chlore, fluor) ; un groupement hydroxy (-OH) ; sulfonique (-SO₃⁻) ; sulfate (-SO₄⁻) ; phosphate (-PO₄H₂) ; amine primaire (-NH₂) ; amine secondaire (-NHR₁), tertiaire (-NR₁R₂) et/ou quaternaire (-N⁺R₁R₂R₃) avec R₁, R₂ et R₃ étant, indépendamment l'un de l'autre, un radical hydrocarboné, linéaire ou ramifié, saturé ou insaturé ayant 1 à 6 atomes de carbone, sous réserve que la somme des atomes de carbone de R₄ + R₅ + R₁ + R₂ + R₃ ne dépasse pas 7 ;
   - l'anhydride maléique ;
   - l'acide itaconique ;
   - l'alcool vinylique de formule CH₂=CHOH ;
   - l'acétate de vinyle de formule CH₂=CH-OCOCH₃ ;
   - les N-vinyllactames tels que la N-vinylpyrrolidone, la N-vinylcaprolactame et la N-butyrolactame ;
   - les vinyléthers de formule CH₂=CHOR₆ dans laquelle R₆ est un radical hydrocarboné, linéaire ou ramifié, saturé ou insaturé, ayant de 1 à 6 carbones ;
   - les dérivés hydrosolubles du styrène, notamment le styrène sulfonate ;
   - le chlorure de diméthyldiallyl ammonium ; et
   - la vinylacétamide.

Les polycondensats et les polymères naturels ou naturels modifiés qui peuvent constituer tout ou partie des unités hydrosolubles sont choisis parmi un ou plusieurs des composants suivants :
- les polyuréthanes hydrosolubles,
- la gomme de xanthane, notamment celle commercialisée sous les dénominations Keltrol T et Keltrol SF par Kelco ; ou Rhodigel SM et Rhodigel 200 de Rhodia ;
- les alginates (Kelcosol de Monsanto) et leurs dérivés tels que l'alginate de propylène glycol (Kelcoloid LVF de Kelco) ;
- les dérivés de cellulose et notamment la carboxyméthylcellulose (Aquasorb A500, Hercules), l'hydroxypropylcellulose, l'hydroxyéthylcellulose et l'hydroxyéthylcellulose quaternisée ;
- les galactomannanes et leurs dérivés, tels que la gomme Konjac, la gomme de guar, l'hydroxypropylguar, l'hydroxypropylguar modifié par des groupements méthylcarboxylate de sodium (Jaguar XC97-1, Rhodia), le chlorure de guar hydroxypropyl triméthyl ammonium.

On peut également citer la polyéthylène imine.

De préférence, les unités hydrosolubles- ont une masse molaire allant de 1 000 g/mole à 5 000 000 g/mole lorsqu'elles constituent le squelette hydrosoluble d'un polymère greffé.

Ces unités hydrosolubles ont de préférence une masse molaire allant de 500 g/mole à 100 000 g/mole lorsqu'elles constituent un bloc d'un polymère multiblocs.

On peut définir les unités à LCST des polymères utilisés dans l'invention, comme étant des unités dont la solubilité dans l'eau est modifiée au-delà d'une certaine température. Il s'agit d'unités présentant une température de démixtion par chauffage (ou point de trouble) définissant leur zone de solubilité dans l'eau. La température de démixtion minimale obtenue en fonction de la concentration en polymère est appelée "LCST" (Lower Critical Solution Température). Pour chaque concentration en polymère, une température de démixtion par chauffage est observée ; elle est supérieure à la LCST qui est le point minimum de la courbe. En dessous de cette température, le polymère constituant l'unité à LCST est soluble dans l'eau ; au-dessus de cette température, le polymère constituant l'unité à LCST perd sa solubilité dans l'eau.

Certains de ces polymères à LCST sont notamment décrits dans les articles de TAYLOR et al., Journal of Polymer Science, part A : Polymer Chemistry, 1975, 13, 2 551 [11] ; de J. BAILEY et al., Journal of Applied Polymer Science, 1959, 1,56 [12] ; et de HESKINS et al., Journal of Macromolecular Science, Chemistry A2, 1968, 1 441 [13].

Par soluble dans l'eau à la température T, on entend que les unités présentent une solubilité à T, d'au moins 1 g/l, de préférence d'au moins 2 g/l.

La mesure de la LCST peut se faire visuellement : on détermine la température à laquelle apparaît le point de trouble de la solution aqueuse ; ce point de trouble se traduit par l'opacification de la solution, ou perte de transparence.

D'une manière générale, une composition transparente aura une valeur de transmittance maximum de la lumière, quelle que soit la longueur d'onde comprise entre 400 et 800 nm, à travers un échantillon de 1 cm d'épaisseur, d'au moins 85 %, de préférence d'au moins 90 %.

La transmittance peut être mesurée en plaçant un échantillon de 1 cm d'épaisseur dans le rayon lumineux d'un spectrophotomètre travaillant dans les longueurs d'onde du spectre lumineux.

Les unités à LCST des polymères utilisés dans l'invention peuvent être constituées par un ou plusieurs polymères choisis parmi les polymères suivants :
- les polyéthers tels que le poly oxyde d'éthylène (POE), le poly oxyde de propylène (POP), les copolymères statistiques d'oxyde d'éthylène (OE) et d'oxyde de propylène (OP),
- le polyvinylméthyléther,
- les dérivés polymériques ou copolymériques N-substitués de l'acrylamide ayant des unités à LCST tels que le poly N-isopropylacrylamide (Nipam) et le poly N-éthylacrylamide ; et
- le polyvinylcaprolactame et les copolymères de vinyl caprolactame.

De préférence, les unités à LCST sont constituées de polyoxyde de propylène (POP)ₙ où n est un nombre entier de 10 à 50 ou de copolymères statistiques d'oxyde d'éthylène (OE) et d'oxyde de propylène (OP), représentés par la formule :

(OE)ₘ (OP)ₙ

dans laquelle m est un nombre entier allant de 1 à 40, de préférence de 2 à 20, et n est un nombre entier allant de 10 à 60, de préférence de 20 à 50.

De préférence, la masse molaire de ces unités à LCST est de 500 à 5 300 g/mole, de préférence encore de 1 500 à 4 000 g/mole.

On a constaté que la répartition statistique des motifs OE et OP se traduit par l'existence d'une température inférieure critique de démixtion, au-delà de laquelle une séparation de phases macroscopique est observée. Ce comportement est différent de celui des copolymères (OE) (OP) à blocs, qui micellisent au-delà d'une température critique dite de micellisation (agrégation à l'échelle microscopique).

Les unités à LCST peuvent donc notamment être issues de copolymères statistiques d'oxyde d'éthylène et d'oxyde de propylène, aminés, notamment monoaminés, diaminés ou triaminés qui sont les copolymères précurseurs de ces unités à LCST. Des précurseurs sont porteurs de sites réactifs, dans ce cas dé groupes aminés, réagissant avec les sites réactifs des précurseurs hydrosolubles, par exemple des groupes carboxyle, pour donner le polymère final mis en oeuvre dans l'invention. Dans le polymère final, les unités hydrosolubles sont liés aux unités à LCST par des groupes de liaison issus de la réaction des groupes ou sites réactifs portés respectivement par les précurseurs des unités à LCST et les précurseurs des unités hydrosolubles. Ces groupes de liaison seront, par exemple, des groupes amide, ester, éther ou uréthane.

Parmi ces polymères précurseurs dont sont issus les unités à LCST, commercialement disponibles, on peut citer les copolymères vendus sous la dénomination Jeffamine par HUNTSMAN, et notamment la Jeffamine XTJ-507 (M-2005), la Jeffamine D-2000 et la Jeffamine XTJ-509 (ou T-3000).

Les unités à LCST peuvent également être issues de copolymères précurseurs OE/OP statistiques à extrémités OH, tels que ceux vendus sous la dénomination Polyglycols P41 et B11 par Clariant.

On peut aussi utiliser dans l'invention comme unités à LCST, les dérivés polymériques et copolymériques N-substitués de l'acrylamide à unités LCST, ainsi que le polyvinyl caprolactame et les copolymères de vinylcaprolactame.

A titre d'exemples de dérivés polymériques et copolymériques N-substitués de l'acrylamide à unités LCST, on peut citer le poly N-isopropylacrylamide, le poly N-éthylacrylamide et les copolymères de N-isopropylacrylamide (ou de N-éthylac-rylamide) et d'un monomère vinylique choisi parmi les monomères ayant la formule (I) donnée ci-dessus, l'anhydride maléique, l'acide itaconique, la vinylpyrrolidone, le styrène et ses dérivés, le chlorure de diméthyldiallyl ammonium, la vinylacétamide, les vinyléthers et les dérivés de l'acétate de vinyle.

La masse molaire de ces polymères est de préférence de 1 000 g/mole à 50 000 g/mole, de préférence de 2 000 à 50 000 g/mole.

La synthèse de ces polymères peut être réalisée par polymérisation radicalaire à l'aide d'un couple d'amorceurs tel que le chlorhydrate d'aminoéthanethiol, en présence de persulfate de potassium, afin d'obtenir des oligomères précurseurs ayant une extrémité réactive aminée.

A titre d'exemples de copolymères de vinylcaprolactame, on peut citer les copolymères de vinyl caprolactame et d'un monomère vinylique ayant la formule (I) donnée ci-dessus, ou d'un monomère choisi parmi l'anhydride maléique, l'acide itaconique, la vinylpyrrolidone, le styrène et ses dérivés, le chlorure de diméthyldiallyl ammonium, la vinylacétamide, l'alcool vinylique, l'acétate de vinyle, les vinyléthers et les dérivés de l'acétate de vinyle.

La masse molaire de ces polymères ou copolymères de vinylcaprolactame est généralement de 1 000 g/mole à 500 000 g/mole, de préférence de 2 000 à 50 000 g/mole.

La synthèse de ces composés peut être réalisée par polymérisation radicalaire à l'aide d'un couple d'amorceurs tel que le chlorhydrate d'aminoéthanethiol, en présence de persulfate de potassium, afin d'obtenir des oligomères précurseurs des unités à LCST ayant une extrémité réactive aminée.

La proportion massique des unités à LCST dans le polymère final est de 5 % à 70 %, notamment de 20 % à 65 %, et particulièrement de 30 % à 60 % en poids, par rapport au polymère final.

On a vu plus haut que la température de démixtion par chauffage desdites unités à LCST du polymère utilisé dans l'invention est de 5 à 40°C, de préférence de 10 à 35°C, pour une concentration massique dans l'eau, de 1 % à 25% en poids desdites unités à LCST.

Les polymères employés dans le cadre de l'invention peuvent être aisément préparés par l'homme du métier sur la base de ses connaissances générales, en utilisant des procédés de greffage, de copolymérisation ou de réaction de couplage.

Lorsque le polymère final se présente sous la forme d'un polymère greffé, notamment présentant un squelette hydrosoluble avec des chaînes latérales ou greffons à LCST, il est possible de le préparer par greffage des précurseurs des unités à LCST ayant au moins une extrémité réactive ou site réactif, notamment aminé(e), sur un polymère hydrosoluble précurseur formant le squelette, portant au minimum 10 % (en mole) de groupes réactifs tels que des fonctions acides carboxyliques. Cette réaction peut s'effectuer en présence d'un carbodiimide tel que le dicyclohexylcarbodiimide ou le chlorhydrate de 1-(3-diméthylaminopropyl)-3-éthyl carbodiimide, dans un solvant tel que la N-méthylpyrrolidone ou l'eau.

Une autre possibilité pour préparer des polymères greffés consiste à copolymériser par exemple un macromonomère à LCST (chaîne à LCST précédemment décrite avec une extrémité vinylique) et un monomère vinylique hydrosoluble tel que l'acide acrylique ou les monomères vinyliques ayant la formule (I).

Lorsque le polymère final se présente sous la forme d'un polymère séquencé ou à blocs, il est possible de le préparer par couplage entre des précurseurs des unités hydrosolubles et des précurseurs des unités à LCST, ces précurseurs ayant à chaque extrémité des sites réactifs complémentaires.

Dans le cas des procédés de greffage et des procédés de couplage, les sites réactifs des précurseurs des unités à LCST peuvent être des fonctions amines notamment monoamines, diamines ou triamines, et des fonctions OH. Dans ce cas, les sites réactifs des précurseurs des unités hydrosolubles peuvent être des fonctions acides carboxyliques. Les groupes liant les unités hydrosolubles et les unités à LCST seront donc, par exemple, des groupes amide ou des groupes ester.

Comme on l'a vu précédemment, les compositions à effet optique de l'invention comprennent une phase aqueuse.

Selon l'invention, la phase aqueuse comprend un polymère comprenant des unités hydrosolubles et des unités à LCST, tel que défini ci-dessus.

Généralement, la concentration massique en polymère de la phase aqueuse est de 0,1 à 20 %, de préférence de 0,5 à 10 %.

En outre, la phase aqueuse de la composition selon l'invention comprend au moins un composé à effet optique. On entend dans la présente invention par "composé à effet optique" un composé qui confère à la composition, des propriétés optiques (par exemple matité, couleur, couvrance) lors de l'application sur la peau.

Ce composé à effet optique peut être choisi notamment parmi les charges, les pigments, les nacres, les polymères matifiants et leurs mélanges.

Par charges, il faut comprendre des particules incolores ou blanches, minérales ou de synthèse, lamellaires ou non lamellaires, destinées à donner du corps ou de la rigidité à la composition et/ou de la douceur, de la matité et de l'uniformité au maquillage. Comme charges, on peut notamment citer le talc, le mica, la silice, le nitrure de bore, l'oxychlorure de bismuth, le kaolin, les poudres de Nylon telles que Nylon-12 (Orgasol commercialisé par la société Atochem), les poudres de polyéthylène, le Téflon (poudres de polymères de tétrafluoroéthylène), les poudres de polyuréthanne, les poudres de polystyrène, les poudres de polyester, l'amidon éventuellement modifié, des microsphères de copolymères, telles que celles commercialisées sous les dénominations Expancel par la société Nobel Industrie, les microéponges comme le Polytrap commercialisé par la société Dow Corning, les microbilles de résine de silicone telles que celles commercialisées par la société Toshiba sous la dénomination Tospearl, le carbonate de calcium précipité, le carbonate et l'hydrocarbonate de magnésium, l'hydroxyapatite, les microsphères de silice creuses (Silica Beads de la Société Maprecos), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium, et leurs mélanges.'

Par pigments, il faut comprendre des particules blanches ou colorées, minérales ou organiques, insolubles dans le milieu, destinées à colorer et/ou opacifier la composition. Ils peuvent être blancs ou colorés, minéraux et/ou organiques, de taille usuelle ou nanométrique. On peut citer, parmi les pigments et les nanopigments minéraux, les dioxydes de titane, de zirconium ou de cérium, ainsi que les oxydes de zinc, de fer ou de chrome, les nanotitanes (nanopigments de dioxyde de titane), les nanozincs (nanopigments d'oxyde de zinc), le bleu ferrique. Parmi les pigments organiques, on peut citer le noir de carbone, et les laques comme les sels de calcium, de baryum, d'aluminium ou de zirconium, de colorants acides tels que les colorants halogéno-acides, azoïques ou anthraquinoniques.

Par nacres, il faut comprendre des particules irisées qui réfléchissent la lumière. Parmi les nacres envisageables, on peut citer la nacre naturelle, le mica recouvert d'oxyde de titane, d'oxyde de fer, de pigment naturel ou d'oxychlorure de bismuth ainsi que le mica titane coloré.

Par polymères matifiants, on entend ici tout polymère en solution, en dispersion ou sous forme de particules, qui empêche la peau de briller et qui unifie le teint. On peut citer par exemple les élastomères de silicone ; les particules de résine ; et leurs mélanges.

Comme élastomères de silicone, on peut citer par exemple les produits commercialisés sous les dénominations « KSG » par la société Shin-Etsu, sous les dénominations « Trefil », « BY29 » ou « EPSX » par la société Dow Corning ou, sous les dénominations « Gransil » par la société Grant Industries.

Comme particules de résine, on peut citer par exemple celles de résine de mélamine-formaldéhyde ou d'urée-formaldéhyde décrites dans le document EP-A-1,046,388.

La concentration massique dans la phase aqueuse de ces charges et/ou pigments et/ou nacres est généralement de 0,1 à 20%, et de préférence de 0,2 à 15% en poids par rapport au poids total de la composition.

La composition à effet optique selon l'invention peut ne comprendre essentiellement que ladite phase aqueuse, elle se présentera alors sous la forme d'une dispersion de charges et/ou de pigments et/ou de nacres contenant le polymère défini plus haut.

Toutefois, généralement, la composition à effet optique selon l'invention se présente sous la forme d'une émulsion huile dans l'eau comprenant ladite phase aqueuse et une phase huileuse dispersée, de préférence uniformément, dans ladite phase aqueuse comprenant un polymère comprenant des unités hydrosolubles et des unités à LCST tel que défini ci-dessus. La concentration massique en polymère de cette phase aqueuse de l'émulsion est celle donnée plus haut.

La phase huileuse de l'émulsion comporte au moins un corps gras et de préférence au moins une huile.

Comme huiles utilisables dans la composition de l'invention, on peut citer par exemple :
- les huiles hydrocarbonées d'origine animale, telles que le perhydrosqualène ;
- les huiles hydrocarbonées d'origine végétale, telles que les triglycérides liquides d'acides gras comportant de 4 à 10 atomes de carbone comme les triglycérides des acides heptanoïque ou octanoïque ou encore, par exemple les huiles de tournesol, de mais, de soja, de courge, de pépins de raisin, de sésame, de noisette, d'abricot, de macadamia, d'arara, de tournesol, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel, l'huile de jojoba, l'huile de beurre de karité;
- les esters et les éthers de synthèse, notamment d'acides gras, comme les huiles de formules R¹COOR² et R¹OR² dans laquelle R¹ représente le reste d'un acide gras comportant de 8 à 29 atomes de carbone, et R² représente une chaîne hydrocarbonée, ramifiée ou non, contenant de 3 à 30 atomes de carbone, comme par exemple l'huile de Purcellin, l'isononanoate d'isononyle, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéarylmalate, le citrate de triisocétyle, les heptanoates, octanoates, décanoates d'alcools gras ; les esters de polyol, comme le dioctanoate de propylène glycol, le diheptanoate de néopentylglycol et le diisononanoate de diéthylèneglycol ; et les esters du pentaérythritol comme le tétraisostéarate de pentaérythrityle ;
- les hydrocarbures linéaires où ramifiés, d'origine minérale ou synthétique, tels que les huiles de paraffine, volatiles ou non, et leurs dérives, la vaseline, les polydécènes, le polyisobutène hydrogéné tel que l'huile de parléam ;
- des huiles essentielles naturelles ou synthétiques telles que, par exemple, les huiles d'eucalyptus, de lavandin, de lavande, de vétiver, de litsea cubeba, de citron, de santal, de romarin, de camomille, de sarriette, de noix de muscade, de cannelle, d'hysope, de carvi, d'orange, de géraniol, de cade et de bergamote;
- les alcools gras ayant de 8 à 26 atomes de carbone, comme l'alcool cétylique, l'alcool stéarylique et leur mélange (alcool cétylstéarylique), l'octyl dodécanol, le 2-butyloctanol, le 2-hexyldécanol, le 2-undécylpentadécanol, l'alcool oléique ou l'alcool linoléique ;
- les huiles fluorées partiellement hydrocarbonées et/ou siliconées comme celles décrites dans le document JP-A-2-295912 ;
- les huiles de silicone comme les polyméthylsiloxanes (PDMS) volatiles ou non à chaîne siliconée linéaire ou cyclique, liquides ou pâteux à température ambiante, notamment les cyclopolydiméthylsiloxanes (cyclométhicones) telles que la cyclohexasiloxane ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphényl-siloxanes, les diphényldiméthicones, les diphénylméthyldiphényl trisiloxanes, les 2-phényléthyltriméthyl-siloxysilicates, et les polyméthylphénylsiloxanes ;
- leurs mélanges.

On entend par « huile hydrocarbonée » dans la liste des huiles citées ci-dessus, toute huile comportant majoritairement des atomes de carbone et d'hydrogène, et éventuellement des groupements ester, éther, fluoré, acide carboxylique et/ou alcool.

Les autres corps gras pouvant être présents dans la phase huileuse sont par exemple les acides gras comportant de 8 à 30 atomes de carbone, comme l'acide stéarique, l'acide laurique, l'acide palmitique et l'acide oléique ; les cires comme la lanoline, la cire d'abeille, la cire de Carnauba ou de Candellila, les cires de paraffine, de lignite ou les cires microcristallines, la cérésine ou l'ozokérite, les cires synthétiques comme les cires de polyéthylène, les cires de Fischer-Tropsch ; les gommes telles que les gommes de silicone (diméthiconol) ; les résines de silicone telles que la trifluorométhyl-C1-4-alkyldimethicone et la trifluoropropyldimethicone.

Ces corps gras peuvent être choisis de manière variée par l'homme du métier afin de préparer une composition ayant les propriétés, par exemple de consistance ou de texture, souhaitées.

La quantité de phase huileuse dans l'émulsion peut aller par exemple de 0,01 à 50 % en poids et de préférence de 0,1 à 30 % en poids par rapport au poids total de la composition.

Quand la composition est sous forme d'émulsions, elle peut contenir avantageusement un tensioactif émulsionnant.

Comme tensio-actifs émulsionnant, on peut citer notamment des émulsionnants non-ioniques et par exemple les produits d'addition de 1 à 200 moles d'oxyde d'éthylène ou d'oxyde de propylène sur des esters partiels de polyols ayant 2 à 16 atomes de carbone et d'acides gras ayant 12 à 22 atomes de carbone comme les esters d'acide gras et de polyéthylène glycol tels que le stéarate de PEG-100, le stéarate de PEG-50 et le stéarate de PEG-40 ; les esters d'acide gras et de polyol tels que le stéarate de glycéryle, le tristéarate de sorbitane et les stéarates de sorbitane oxyéthylénés disponibles sous les dénominations commerciales Tween® 20 ou Tween® 60, les esters de sucre comme le stéarate de sucrose, et leurs mélanges.

La phase aqueuse peut éventuellement, en outre, comprendre un agent gélifiant à une concentration massique de 0,01 à 5%, de préférence de 0,01 à 3% en poids par rapport au poids total de la composition.

Dans les compositions à effet optique de l'invention, la phase aqueuse est constituée de préférence d'un milieu physiologiquement acceptable permettant une application topique, et notamment cosmétique.

On entend dans la présente demande par "milieu physiologiquement acceptable" un milieu compatible avec toutes les matières kératiniques telles que la peau y compris le cuir chevelu, les ongles, les muqueuses, les yeux et les cheveux ou toute autre zone cutanée du corps.

Le milieu physiologiquement acceptable des compositions à effet optique de l'invention comprend de l'eau. La quantité d'eau peut aller de 30 à 99,98% en poids et de préférence de 40 à 95 % en poids par rapport au poids total de la composition.

L'eau utilisée peut être outre de l'eau, une eau florale telle que l'eau de bleuet, une eau minérale telle que l'eau de Vittel, l'eau de LUCAS ou l'eau de la Roche Posay et/ou une eau thermale.

Le milieu physiologiquement acceptable peut contenir, outre l'eau, un ou plusieurs solvants choisis parmi les alcools inférieurs comportant de 1 à 8 atomes de carbone, tels que l'éthanol ; les polyols tels que la glycérine ; les glycols comme le butylène glycol, l'isoprène glycol, le propylène glycol, les polyéthylène glycols tels que le PEG-8 ; le sorbitol ; les sucres tels que le glucose, le fructose, le maltose, le lactose et le sucrose ; et leurs mélanges. La quantité de solvant(s) peut aller de 0,5 à 30 % en poids et de préférence de 5 à 20 % en poids par rapport au poids total de la phase aqueuse.

Les compositions à effet optique de l'invention peuvent encore contenir des adjuvants habituels dans les domaines cosmétique et dermatologique, tels que les actifs hydrophiles ou lipophiles, les conservateurs, les gélifiants, les plastifiants, les antioxydants, les parfums, les absorbeurs d'odeur, les agents antimousse, les filtres UV, les séquestrants (EDTA), les ajusteurs de pH acides ou basiques ou des tampons, et des colorants.

Les quantités de ces différents additifs sont celles classiquement utilisées dans les domaines considérés, et par exemple de 0,01 à 20 % du poids total de la composition. Bien entendu, l'homme de l'art veillera à choisir le ou les éventuels composés à ajouter aux compositions moussantes selon l'invention de manière telle que les propriétés avantageuses attachées intrinsèquement à ces compositions ne soient pas, ou substantiellement pas, altérées par l'addition envisagée.

Comme on l'a déjà indiqué plus haut, on peut ajouter aux compositions de l'invention un agent gélifiant afin de régler la texture de la composition et d'accéder à une large gamme de textures allant du lait à la crème. Comme on l'a déjà mentionné plus haut, les compositions selon l'invention peuvent à faible température, par exemple à température ambiante, « dans le pot », revêtir toute texture souhaitable. Il n'y a aucune restriction sur la texture que la composition peut avoir, avant application. En particulier, la composition ne doit pas contenir obligatoirement un agent gélifiant pour que lors de l'application, on obtienne une texture gélifiée.

En effet, grâce au polymère spécifique inclus dans la composition de l'invention, on obtient, lors de l'augmentation de température se produisant, par exemple, lors de l'application de la composition, notamment sur la peau une texture gélifiée, stable, quelle que soit la texture, la forme de la composition à effet optique avant application, "dans le pot". On inclura donc un gélifiant dans la composition que si l'on souhaite que celle-ci ait un aspect gélifié, cet aspect n'étant selon l'invention qu'un aspect particulier parmi la multitude d'aspects, de textures, de formes que peut avoir la composition à effet optique.

Les gélifiants utilisables peuvent être des gélifiants hydrophiles. A titre d'exemples de gélifiants hydrophiles, on peut citer en particulier les polymères carboxyvinyliques (carbomer), les copolymères acryliques tels que les copolymères d'acrylates/alkylacrylates, les polyacrylamides, les polysaccharides, les gommes naturelles et les argiles.

Les compositions à effet optique de l'invention peuvent se présenter notamment sous la forme d'une composition cosmétique, de maquillage ou de soin, susceptible d'être appliquée sur la peau y compris le cuir chevelu, les ongles, les cheveux, les cils, les sourcils, les yeux, les muqueuses et les semi-muqueuses, et toute autre zone cutanée du corps et du visage.

Ces compositions à effet optique peuvent, de manière générale, être définies comme des produits tels que des produits de maquillage ou de soin apportant après application une modification de l'aspect du visage ou du corps appréciable de façon visuelle, à l'oeil nu. Par aspect, on entend aussi bien la couleur que l'aspect de surface, que le relief, etc...

A titre d'exemples de telles compositions, on peut citer les fonds de teint, les fards à joues ou à paupières, les mascaras, les produits apportant de la couvrance ou du "soft focus", les produits solaires, les produits capillaires.

Rappelons qu'une composition apportant du "soft focus" est une composition capable d'estomper les défauts de la peau, tels que les microreliefs et les variations de coloration tout en conservant à celle-ci un aspect naturel dû essentiellement au caractère translucide de cette couche mince, c'est-à-dire de rendre moins visibles les variations de couleur et les microreliefs cutanés, les pores et les ridules.

Aussi, un autre objet de l'invention consiste en l'utilisation cosmétique de la composition telle que définie précédemment, pour estomper les imperfections du relief de la peau et/ou pour camoufler les microreliefs, les rides, les ridules et/ou les pores de la peau.

L'invention a aussi pour objet l'utilisation cosmétique de la composition telle que définie précédemment, pour le maquillage de la peau, des cils, des lèvres et/ou des cheveux.

L'invention concerne également un procédé de traitement cosmétique de la peau destiné à lui apporter un aspect mat et/ou à camoufler les défauts du relief de la peau, caractérisé par le fait qu'on applique sur la peau une composition telle que définie précédemment.

D'autres caractéristiques et avantages de l'invention apparaîtront mieux à la lecture de la description qui suit donnée à titre illustratif et non limitatif.

### Description détaillée des modes de réalisation

Les exemples suivants illustrent la réalisation de compositions cosmétiques à effet optique selon l'invention comprenant des polymères comportant des unités hydrosolubles et des unités à LCST spécifiques.

Le polymère utilisé dans ces exemples est constitué par un squelette d'acide polyacrylique (PAA) portant des chaînes latérales ou greffons constituées par des unités à LCST spécifiques. Il est caractérisé par la masse molaire du squelette hydrosoluble (acide polyacrylique), la nature chimique des chaînes à LCST, leur proportion massique dans le polymère et leur masse molaire.

On compare également les propriétés du polymère de l'invention avec celles d'un acide polyacrylique pris à titre de polymère référence, comparatif, représentatif des polymères ne portant pas de chaînes latérales à LCST, qui sont les polymères inclus dans les compositions cosmétiques à effet optique de l'art antérieur.

Les caractéristiques du polymère selon l'invention et du polymère de référence (comparatif) utilisés sont données dans le tableau I.

**Tableau I**

| | Squelette hydrosoluble | Greffons (unités à LCST) | Proportion : unités à LCST dans polymère final (en poids) | Taux de greffage (% en mole) |
|---|---|---|---|---|
| Polymère 1 selon l'invention | Acide polyacrylique; PM=450 000 | (OE)₆(OP)₃₉ statistique Jeffamine M-2005; PM=2 600 | 51 % | 3,9% |
| Polymère 2 de référence | Acide polyacrylique; PM=450 000 | / | 0 % | 0 % |

Ces polymères sont préparés de la façon suivante.

### Préparation du Polymère 1 selon l'invention

Dans un réacteur de 500 ml muni d'un réfrigérant, on dissout 3 g d'acide polyacrylique de masse molaire moyenne 450 000 g/mole (Aldrich) dans 220 ml de N-méthyl pyrrolidone sous agitation à 60°C pendant 12 h.

On dissout 4,181 g de copolymère (OE)₆(OP)₃₉ statistique monoaminé, de masse molaire 2600 g/mole ayant un point de trouble, à une concentration de 1% en poids dans l'eau, de 16°C (Jeffamine M-2005 de Huntsman) dans 50 ml de N-méthylpyrrolidone sous agitation, à 20°C, pendant 15 min.. La solution obtenue est ajoutée goutte à goutte au milieu réactionnel contenant l'acide polyacrylique, sous vive agitation à 60°C.

On dissout 2,158 g de dicyclohexylcarbodiimide dans 30 ml de N-méthylpyrrolidone sous agitation à 20°C pendant 15 min.. La solution obtenue est ajoutée goutte à goutte au milieu réactionnel contenant l'acide polyacrylique et le copolymère (OE)₆(OP)₃₉ statistique monoaminé, sous vive agitation à 60°C. On agite le mélange final pendant 12 h à 60°C.

On refroidit le mélange à 20°C puis on le place dans un réfrigérateur à 4°C pendant 24 h. Les cristaux de dicyclohexylurée formés sont éliminés par filtration du milieu réactionnel.

Le polymère est alors neutralisé à l'aide de 19 g de soude à 35 % (4 fois en excès par rapport au nombre de mole d'acide acrylique), ce qui conduit à sa précipitation. Après 12 h au repos, le milieu réactionnel est filtré afin de récupérer le polymère précipité. Celui-ci est séché sous vide à 35°C pendant 24 h.

On récupère 13,55 g de solide qui sont dissous dans 2 1 d'eau désionisée. Cette solution est ultrafiltrée à l'aide d'un système d'ultrafiltration Millipore contenant une membrane dont le seuil de coupure est fixée à 10 000 Daltons. La solution ainsi purifiée est lyophilisée afin de recueillir le polymère sous forme solide.

On obtient 7,05 g d'acide polyacrylique (450 000 g/mole) greffé par 3,9 % (en mole) de copolymère (OE)₆(OP)₃₉ statistique monoaminé.

La proportion massiques des unités à LCST dans le polymère final est de 51 %.

Le polymère ainsi obtenu présente une solubilité dans l'eau, à 20°C, d'au moins 10 g/l.

Le polymère 2 de référence, témoin, comparatif est un polymère disponible auprès de la Société ALDRICH sous la dénomination de Acide acrylique de masse molaire 450 000 g/mole.

### EXEMPLE 1

Dans cet exemple, on étudie la variation du tack (pouvoir collant superficiel) d'un film du polymère d'acide acrylique 2 de référence, en comparaison avec un film de polymère thermogélifiant 1 selon l'invention (polymère acide acrylique à greffon LCST), en fonction de la température et de l'humidité relative (HR).

### Mode opératoire

### 1) Préparation des films de chacun des polymères

Dans un récipient métallique au fond parfaitement rectifié posé sur une surface bien horizontale, on met à sécher à l'abri de la poussière une solution de la matière à tester (eau + polymère) dans un solvant volatil, (l'eau) en concentration telle que la quantité de solution à sécher s'étale bien dans le récipient et que l'épaisseur du dépôt soit de 100 microns.

### 2) Mesure du pouvoir collant superficiel

Après 24h de séchage à température ambiante, on dépose le récipient métallique sur une plaque à circulation d'eau dont le bain thermostaté est réglé à 35°C (la surface du film sera environ à 32°C) pendant une heure minimum.

On installe le palpeur (en alliage d'aluminium, diamètre 6 mm) sur le bras de l'analyseur de texture (TATXT2 de Rheo) et après étalonnage on l'amène à 2 mm environ de la surface à mesurer. Il faut assurer, pendant l'essai, le parallélisme entre les deux surfaces à mettre en contact (film/palpeur). Ceci peut, par exemple, être réalisé en posant le moule sur un ensemble dont l'horizontalité est ajustable. On amène le palpeur à 1 mm/s jusqu'au contact puis on porte la pression du palpeur à 10 000 Pa à la vitesse de 0,1 mm/s. On maintient cette pression pendant 20 s puis on remonte à la position de départ à la vitesse de 0,1 mm/s. Le collant du film est évalué lors de cette phase de retrait par la mesure de l'énergie nécessaire pour séparer les deux surfaces en contact (intégration de la courbe "force en fonction du déplacement").

### Résultats

Collant (énergie de séparation) en J/m².

| Température | 27°C | 30°C | 35°C |
|---|---|---|---|
| Humidité relative (HR) | 80% HR | 30% HR | 80% HR |
| Collant du polymère témoin non greffé | 0,9 ± 0,3 | 0±0,03 | 0,5 ± 0,3 |
| Collant du polymère thermogélifiant | 0 | 0+0,03 | 0+0,03 |

### Conclusions

I1 ressort de cette étude que le collant du polymère 2, référence, témoin non greffé est sensible à l'humidité relative, contrairement au polymère 1 greffé selon l'invention dont le collant reste nul quelles que soient l'humidité relative et la température considérées.

### EXEMPLE 2

Cet exemple concerne une composition de mascara selon l'invention comprenant le polymère 1 conforme à l'invention.

**Mascara**

| | |
|---|---|
| Cire d'abeille | 10% |
| Cire de carnauba | 10% |
| Acide stéarique | 5,6% |
| Triéthanolamine | 3,1% |
| Pigments | 5% |
| Polymère de l'invention | 6% |
| Eau déminéralisée | 60,3% |

Cette composition de mascara conduit à des dépôts ou fils ayant une bonne tenue en atmosphère humide (80% HR).

### EXEMPLE 3

Cet exemple concerne une composition de fond de teint fluide comprenant le polymère 1 conforme à l'invention.

**Fond de teint fluide (émulsion H/E)**

| Phase huileuse | |
|---|---|
| Huile de Parléam | 12% |
| Cyclopentadiméthylsiloxane | 8% |

| Phase aqueuse | |
|---|---|
| Pigments | 10% |
| Polymère de l'invention | 8% |
| Conservateur | 0,2% |
| Eau déminéralisée | 61,8% |

Ce fond de teint fluide conduit à des dépôts ou films ayant une bonne tenue en atmosphère humide (80% HR).

### REFERENCES

**[1]** D. HOURDET et al., Polymer, 1994, vol. 35, n° 12, pages 2624-2630.
**[2]** F. L'ALLORET et al., Coll. Polym. Sci., 1995, vol. 273, n° 12, pages 1 163-1 173.
**[3]** F. L'ALLORET, Revue de l'Institut Français du Pétrole, 1997, vol. 52, n° 2, pages 117-128.
**[4]** EP-A-0 583 814.
**[5]** EP-A-0 629 649.
**[6]** WO-A-95 24430.
**[7]** US-A-5 939 485.
**[8]** WO-A-97 00275.
**[9]** WO-A-98 48768.
**[10]** WO-A-00 35961.
**[11]** Articles de TAYLOR et al., Journal of Polymer Science, part A : Polymer Chemistry, 1975, 13, 2 551.
**[12]** J. BAILEY et al., Journal of Applied Polymer Science, 1959, 1,56.
**[13]** HESKINS et al., Journal of Macromolecular Science, Chemistry A2, 1968, 1 441.

## Revendications

1. Composition cosmétique comprenant une phase aqueuse, ladite phase aqueuse comprenant au moins un composé à effet optique choisi parmi les charges, les pigments, les nacres, les polymères matifiants et leurs mélanges, et un polymère comprenant des unités hydrosolubles et des unités présentant dans l'eau une température inférieure critique de démixtion LCST, la température de démixtion par chauffage en solution aqueuse desdites unités à LCST étant de 5 à 40°C pour une concentration massique dans l'eau de 1 % à 25% desdites unités, et ledit polymère se présentant sous la forme d'un polymère séquencé (ou blocs) comprenant des séquences constituées d'unités hydrosolubles alternées avec des séquences constituées d'unités à LCST, ou sous la forme d'un polymère greffé dont le squelette est formé d'unités hydrosolubles ledit squelette étant porteur de greffons constitués d'unités à LCST ; lesdits polymères pouvant être partiellement réticulés ; et la proportion massique des unités à LCST du polymère est de 5 à 70% par rapport au polymère.

2. Composition cosmétique selon la revendication 1, dans laquelle la température de démixtion par chauffage en solution aqueuse des unités à LCST du polymère est de 10 à 35°C pour une concentration massique dans l'eau de 1 % à 25% desdites unités.

3. Composition cosmétique selon l'une quelconque des revendications précédentes, dans laquelle les unités hydrosolubles sont, en totalité ou en partie, susceptibles d'être obtenues par polymérisation, ou par polycondensation ou encore sont constituées, en totalité ou en partie, par des polymères naturels ou naturels modifiés.

4. Composition cosmétique selon la revendication 3, dans laquelle les unités hydrosolubles sont, en totalité ou en partie, susceptibles d'être obtenues par polymérisation, notamment radicalaire, d'au moins un monomère choisi parmi les monomères suivants :
- l'acide (méth)acrylique ;
- les monomères vinyliques de formule (I) suivante : 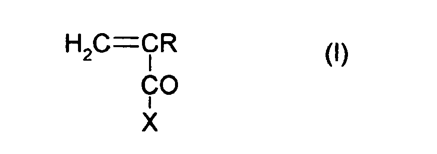 dans laquelle :
• R est choisi parmi H, -CH₃, -C₂H₅ ou -C₃H₇, et
- X est choisi parmi :
• les oxydes d'alkyle de type -OR' où R' est un radical hydrocarboné, linéaire ou ramifié, saturé ou insaturé, ayant de 1 à 6 atomes de carbone, éventuellement substitué par au moins un atome d'halogène (iode, brome, chlore, fluor); un groupement sulfonique (-SO₃⁻), sulfate (-SO₄⁻), phosphate (-PO₄H₂) ; hydroxy (-OH) ; amine primaire (-NH₂) ; amine secondaire (-NHR₁), tertiaire (-NR₁R₂) ou quaternaire (-N⁺R₁R₂R₃) avec R₁, R₂ et R₃ étant, indépendamment l'un de l'autre, un radical hydrocarboné, linéaire ou ramifié, saturé ou insaturé ayant 1 à 6 atomes de carbone, sous réserve que la somme des atomes de carbone de R' + R₁ + R₂ + R₃ ne dépasse pas 7 ; et
• les groupements -NH₂, -NHR₄ et -NR₄R₅ dans lesquels R₄ et R₅ sont indépendamment l'un de l'autre des radicaux hydrocarbonés, linéaires ou ramifiés, saturés ou insaturés ayant 1 à 6 atomes de carbone, sous réserve que le nombre total d'atomes de carbone de R₄ + R₅ ne dépasse pas 7, lesdits R₄ et R₅ étant éventuellement substitués par un atome d'halogène (iode, brome, chlore, fluor) ; un groupement hydroxy (-OH) ; sulfonique (-SO₃⁻) ; sulfate (-SO₄⁻) ; phosphate (-PO₄H₂) ; amine primaire (-NH₂) ; amine secondaire (NHR₁), tertiaire (-NR₁R₂) et/ou quaternaire (-N⁺R₁R₂R₃) avec R₁, R₂ et R₃ étant, indépendamment l'un de l'autre, un radical hydrocarboné, linéaire ou ramifié, saturé ou insaturé ayant 1 à 6 atomes de carbone, sous réserve que la somme des atomes de carbone de R₄ + R₅ + R₁ + R₂ + R₃ ne dépasse pas 7 ;
- l'anhydride maléique ;
- l'acide itaconique ;
- l'alcool vinylique de formule CH₂=CHOH ;
- l'acétate de vinyle de formule CH₂=CH-OCOCH₃ ;
- les N-vinyllactames tels que la N-vinylpyrrolidone, la N-vinylcaprolactame et la N-butyrolactame ;
- les vinyléthers de formule CH₂=CHOR₆ dans laquelle R₆ est un radical hydrocarboné, linéaire ou ramifié, saturé ou insaturé, ayant de 1 à 6 atomes de carbone ;
- les dérivés hydrosolubles du styrène, notamment le styrène sulfonate ;
- le chlorure de diméthyldiallyl ammonium ; et
- la vinylacétamide.

5. Composition cosmétique selon la revendication 3, dans laquelle les unités hydrosolubles du polymère sont constituées en totalité ou en partie par des polycondensats ou par des polymères naturels ou naturels modifiés choisis parmi un ou plusieurs des composants suivants :
- les polyuréthannes hydrosolubles ;
- la gomme de xanthane ;
- les alginates et leurs dérivés tels que l'alginate de propylèneglycol ;
- les dérivés de cellulose et notamment la carboxyméthylcellulose, l'hydroxypropylcellulose, l'hydroxyéthylcellulose et l'hydroxyéthylcellulose quaternisée ;
- les galactomannanes et leurs dérivés tels que la gomme Konjac, la gomme de guar, l'hydroxypropylguar, l'hydroxypropylguar modifié par des groupements méthylcarboxylate de sodium, et le chlorure de guar hydroxypropyl triméthyl ammonium ; et
- la polyéthylène-imine.

6. Composition cosmétique selon l'une quelconque des revendications 4 à 5, dans laquelle les unités hydrosolubles du polymère ont une masse molaire allant de 1 000 g/mole à 5 000 000 g/mole lorsqu'elles constituent le squelette hydrosoluble d'un polymère greffé, ou une masse molaire allant de 500 g/mole à 100 000 g/mole lorsqu'elles constituent un bloc d'un polymère multiblocs.

7. Composition cosmétique selon l'une quelconque des revendications 1 à 6, dans laquelle les unités à LCST du polymère sont constituées d'un ou plusieurs polymères choisis parmi les polymères suivants :
- les polyéthers tels que le polyoxyde d'éthylène (POE), le polyoxyde de propylène (POP), et les copolymères statistiques d'oxyde d'éthylène (OE) et d'oxyde de propylène (OP) ;
- les polyvinylméthyléthers ;
- les dérivés polymériques ou copolymériques N-substitués de l'acrylamide ayant des unités à LCST ; et
- le polyvinylcaprolactame et les copolymères de vinyl caprolactame.

8. Composition cosmétique selon l'une quelconque des revendications 1 à 7, dans laquelle les unités à LCST du polymère sont constituées de polyoxyde de propylène (POP)ₙ où n est un nombre entier de 10 à 50 ou de copolymères statistiques d'oxyde d'éthylène (OE) et d'oxyde de propylène (OP), représentés par la formule :
(OE)ₘ (OP)ₙ
dans laquelle m est un nombre entier allant de 1 à 40, de préférence de 2 à 20, et n est un nombre entier allant de 10 à 60, de préférence de 20 à 50.

9. Composition cosmétique selon la revendication 8, dans laquelle la masse molaire des unités à LCST du polymère est de 500 à 5 300 g/mole, de préférence de 1 500 à 4 000 g/mole.

10. Composition cosmétique selon la revendication 9, dans laquelle les unités à LCST du polymère sont constituées par un dérivé polymérique ou copolymérique N-substitué de l'acrylamide à unité LCST.

11. Composition cosmétique selon la revendication 10, dans laquelle les unités à LCST du polymère sont constituées par un polymère choisi parmi le poly N-isopropylacrylamide, le poly N-éthyl acrylamide et les copolymères de N-isopropylacrylamide ou de N-éthylacrylamide et d'un monomère vinylique choisi parmi les monomères ayant la formule (I) donnée dans la revendication 4, l'anhydride maléique, l'acide itaconique, la vinylpyrrolidone, le styrène et ses dérivés, le chlorure de diméthyldiallyl ammonium, la vinylacétamide, l'alcool vinylique, l'acétate de vinyle, les vinyléthers et les dérivés de l'acétate de vinyle.

12. Composition cosmétique selon la revendication 10 ou 11, dans laquelle la masse molaire des unités à LCST du polymère est de 1 000 g/mole à 500 000 g/mole, de préférence de 2 000 à 50 000 g/mole.

13. Composition cosmétique selon l'une quelconque des revendications 1 à 7, dans lequel les unités à LCST du polymère sont constituées par un polyvinylcaprolactame ou un copolymère de vinylcaprolactame et d'un monomère vinylique choisi parmi les monomères répondant à la formule (I) donnée dans la revendication 4, l'anhydride maléique, l'acide itaconique, la vinylpyrrolidone, le styrène et ses dérivés, le chlorure de diméthyldiallyl ammonium, la vinylacétamide, l'alcool vinylique, l'acétate de vinyle, les vinyléthers et les dérivés de l'acétate de vinyle.

14. Composition cosmétique selon la revendication 13, dans lequel la masse molaire des unités à LCST est de 1 000 à 500 000 g/mole, de préférence de 2 000 à 50 000 g/mole.

15. Composition cosmétique selon l'une quelconque des revendications 1 à 14, dans laquelle la proportion massique des unités à LCST du polymère est de 20 à 65 %, et mieux encore de 30 à 60 %, par rapport au polymère.

16. Composition cosmétique selon l'une quelconque des revendications 1 à 15, dans laquelle la concentration massique en polymère de la phase aqueuse est de 0,1 à 20 %, de préférence de 0,5 à 10%.

17. Composition cosmétique selon l'une quelconque des revendications 1 à 16 qui est une dispersion.

18. Composition cosmétique selon l'une quelconque des revendications 1 à 16 qui est une émulsion huile-dans-l'eau comprenant outre la phase aqueuse, une phase huileuse dispersée dans la phase aqueuse.

19. Composition cosmétique selon la revendication 18, dans laquelle la phase aqueuse comprend, en outre, éventuellement un tensioactif émulsionnant.

20. Composition cosmétique selon l'une quelconque des revendications 1 à 19, dans laquelle la phase aqueuse comprend éventuellement, en outre, un agent gélifiant à une concentration de 0,01 à 5% en poids par rapport au poids total de la composition.

21. Composition cosmétique selon l'une quelconque des revendications 1 à 20, dans laquelle la phase aqueuse est constituée par un milieu physiologiquement acceptable.

22. Composition cosmétique selon l'une quelconque des revendications 1 à 21, se présentant sous la forme d'une composition cosmétique, de maquillage ou de soin, susceptible d'être appliquée sur la peau y compris le cuir chevelu, les ongles, les cheveux, les cils, les sourcils, les yeux, les muqueuses et les semi-muqueuses, et toute autre zone cutanée du corps et du visage.

23. Composition selon l'une quelconque des revendications 1 à 22, qui est choisie parmi les fonds de teint, les fards à joues ou à paupières, les mascaras, les produits apportant de la couvrance ou du "soft focus", les produits solaires, et capillaires.

24. Utilisation du polymère tel que décrit dans la revendication 1, pour annuler ou diminuer le pouvoir collant superficiel d'un film ou dépôt obtenu à partir d'une composition à effet optique contenant ledit polymère.

25. Utilisation du polymère tel que décrit selon la revendication 1 pour maintenir la tenue d'un film ou dépôt obtenu à partir d'une composition à effet optique contenant ledit polymère.

26. Utilisation selon la revendication 23 ou 24, dans laquelle ledit film ou dépôt est exposé à une atmosphère chaude et/ou humide.

27. Utilisation selon la revendication 26, dans laquelle l'humidité relative (HR) de ladite atmosphère est de 40 à 95%.

28. Utilisation selon la revendication 26 ou 27, dans laquelle la température de ladite atmosphère est de 25 à 45°C.

29. Utilisation cosmétique de la composition selon l'une quelconque des revendications 1 à 23, pour estomper les imperfections du relief de la peau et/ou pour camoufler les microreliefs, les rides, les ridules et/ou les pores de la peau.

30. Utilisation cosmétique de la composition selon l'une quelconque des revendications 1 à 23, pour le maquillage de la peau, des cils, des lèvres et/ou des cheveux.

31. Procédé de traitement cosmétique de la peau, destiné à lui apporter un aspect mat et/ou à camoufler les défauts du relief de la peau, **caractérisé par le fait qu'**on applique sur la peau la composition selon l'une quelconque des revendications 1 à 23.

## Claims

1. Cosmetic composition comprising an aqueous phase, said aqueous phase comprising at least one optical effect compound chosen from among fillers, pigments, nacres, matt-effect polymers and their mixtures, and a polymer comprising water-soluble units and units having in water a lower critical solution temperature LCST, the heat-induced demixing temperature in aqueous solution of said LCST units being 5 to 40°C for a mass concentration in water of 1 to 25% of said units, and said polymer being in the form of a sequenced or block polymer comprising sequences constituted by water-soluble units alternating with sequences constituted by LCST units, or in the form of a grafted polymer, whose skeleton is formed by water-soluble units, said skeleton carrying grafts constituted by LCST units, whereby said polymers can be partly crosslinked, and the mass proportion of the LCST units of the polymer is 5 to 70% based on the polymer.

2. Cosmetic composition according to claim 1, in which the heat-induced demixing temperature in aqueous solution of the units with an LCST of the polymer is from 10 to 35°C for a concentration by mass in water of from 1% to 25% of said units.

3. Cosmetic composition according to any one of the preceding claims, in which the water-soluble units are totally or partially obtainable by polymerization or by polycondensation, or alternatively consist totally or partially of natural polymers or modified natural polymers.

4. Cosmetic composition according to claim 3, in which the water-soluble units are totally or partially obtainable by polymerization, especially free-radical polymerization, of at least one monomer chosen from the following monomers:
- (meth)acrylic acid;
- vinyl monomers of formula (I) below: 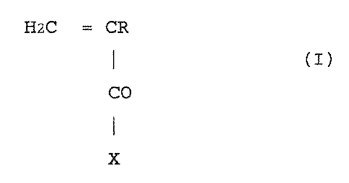
in which:
- R is chosen from H, -CH₃, -C₂H₅ or -C₃H₇; and
- X is chosen from:
- alkyl oxides of -OR' type in which R' is a linear or branched, saturated or unsaturated hydrocarbon-based radical containing from 1 to 6 carbon atoms, optionally substituted with at least one halogen atom (iodine, bromine, chlorine or fluorine); a sulphonic (-SO₃-), sulphate (-SO₄-), phosphate (-PO₄H₂); hydroxyl (-OH); primary amine (-NH₂); secondary amine (-NHR₁), tertiary amine (-NR₁R₂) or quaternary amine (-N⁺R₁R₂R₃) group with R₁, R₂ and R₃ being, independently of each other, a linear or branched, saturated or unsaturated hydrocarbon-based radical containing 1 to 6 carbon atoms, with the proviso that the sum of the carbon atoms of R' + R₁ + R₂ + R₃ does not exceed 7; and
- NH₂, -NHR₄ and -NR₄R₅ groups in which R₄ and R₅ are, independently of each other, linear or branched, saturated or unsaturated hydrocarbon-based radicals containing 1 to 6 carbon atoms, with the proviso that the total number of carbon atoms of R₄ + R₅ does not exceed 7, the said R₄ and R₅ optionally being substituted with a halogen atom (iodine, bromine, chlorine or fluorine); a hydroxyl (-OH); sulphonic (-SO₃⁻) ; sulphate (-SO₄⁻); phosphate (-PO₄H2); primary amine (-NH₂): secondary amine (-NHR₁), tertiary amine (-NR₁R₂) and/or quaternary amine (-N⁺R₁R₂R₃) group with R₁, R₂ and R₃ being, independently of each other, a linear or branched, saturated or unsaturated hydrocarbon-based radical containing 1 to 6 carbon atoms, with the proviso that the sum of the carbon atoms of R₄ + R₅ + R₁ + R₂ + R₃ does not exceed 7;
- maleic anhydride;
- itaconic acid;
- vinyl alcohol of formula CH₂=CHOH;
- vinyl acetate of formula CH₂=CH-OCOCH₃;
- N-vinyllactams such as N-vinylpyrrolidone, N-vinylcaprolactam and N-butyrolactam;
- vinyl ethers of formula CHOR₆ in which R₆ is a linear or branched, saturated or unsaturated hydrocarbon-based radical containing from 1 to 6 carbon atoms;
- water-soluble styrene derivatives, especially styrene sulphonate;
- dimethyldiallylammonium chloride; and
- vinylacetamide.

5. Cosmetic composition according to claim 4, in which the water-soluble units of the polymer consist totally or partially of polycondensates or of natural polymers or modified natural polymers chosen from one or more of the following components:
- water-soluble polyurethanes;
- xanthan gum;
- alginates and derivatives thereof such as propylene glycol alginate;
- cellulose derivatives and especially carboxymethylcellulose, hydroxypropylcellulose, hydroxyethylcellulose and quaternized hydroxyethylcellulose;
- galactomannans and derivatives thereof, such as Konjac gum, guar gum, hydroxypropylguar, hydroxypropylguar modified with sodium methylcarboxylate groups, and hydroxypropyltrimethylammonium guar chloride; and
- polyethyleneimine.

6. Cosmetic composition according to either of claims 4 and 5, in which the water-soluble units of the polymer have a molar mass ranging from 1000 g/mol to 5,000,000 g/mol when they constitute the water-soluble backbone of a grafted polymer, or a molar mass ranging from 500 g/mol to 100,000 g/mol when they constitute a block of a multiblock polymer.

7. Cosmetic composition according to any one of claims 1 to 6, in which the units with an LCST of the polymer consist of one or more polymers chosen from the following polymers:
- polyethers such as polyethylene oxide (PEO), polypropylene oxide (PPO) or random copolymers of ethylene oxide (EO) and of propylene oxide (PO);
- polyvinyl methyl ethers;
- polymeric and copolymeric N-substituted acrylamide derivatives containing units with an LCST; and
- polyvinylcaprolactam and vinylcaprolactam copolymers.

8. Cosmetic composition according to any one of claims 1 to 7, in which the units with an LCST of the polymer consist of polypropylene oxide (PPO)n where n is an integer from 10 to 50, or of random copolymers of ethylene oxide (EO) and of propylene oxide (PO), represented by the formula:
(EO)ₘ(PO)ₙ
in which m is an integer ranging from 1 to 40 and preferably from 2 to 20, and n is an integer ranging from 10 to 60 and preferably from 20 to 50.

9. Cosmetic composition according to claim 8, in which the molar mass of the units with an LCST of the polymer is from 500 to 5300 g/mol, preferably from 1500 to 4000 g/mol.

10. Cosmetic composition according to claim 9, in which the units with LCST of the polymer consist of a polymeric or copolymeric N-substituted acrylamide derivative containing units with an LCST.

11. Cosmetic composition according to claim 10, in which the units with an LCST of the polymer consist of a polymer chosen from poly-N-isopropylacrylamide, poly-N-ethylacrylamide and copolymers of N-isopropylacrylamide or of N-ethylacrylamide and of a vinyl monomer chosen from the monomers having the formula (I) given in claim 5, maleic anhydride, itaconic acid, vinylpyrrolidone, styrene and its derivatives, dimethyldiallylammonium chloride, vinylacetamide, vinyl alcohol, vinyl acetate, vinyl ethers and vinyl acetate derivatives.

12. Cosmetic composition according to claim 10 or 11, in which the molar mass of the units with an LCST of the polymer is from 1000 g/mol to 500,000 g/mol and preferably from 2000 to 50,000 g/mol.

13. Cosmetic composition according to any one of claims 1 to 7, in which the units with an LCST of the polymer consist of a polyvinyl caprolactam or a copolymer of vinylcaprolactam and of a vinyl monomer chosen from the monomers corresponding to formula (I) given in claim 5, maleic anhydride, itaconic acid, vinylpyrrolidone, styrene and its derivatives, dimethyldiallylammonium chloride, vinylacetamide, vinyl alcohol, vinyl acetate, vinyl ethers and vinyl acetate derivatives.

14. Cosmetic composition according to claim 13, in which the molar mass of the units with an LCST is from 1000 to 500,000 g/mol and preferably from 2000 to 50,000 g/mol.

15. Cosmetic composition according to any one of claims 1 to 14, in which the proportion by mass of the units with an LCST of the polymer is from 5 to 70%, preferably from 20 to 65% and better still from 30 to 60% relative to the polymer.

16. Cosmetic composition according to any one of claims 1 to 15, in which the concentration by mass of polymer in the aqueous phase is from 0.1 to 20% and preferably from 0.5 to 10%.

17. Cosmetic composition according to any one of claims 1 to 16, which is a dispersion.

18. Cosmetic composition according to any one of claims 1 to 16, which is an oil-in-water emulsion comprising, in addition to the aqueous phase, an oily phase dispersed in the aqueous phase.

19. Cosmetic composition according to claim 18, in which the aqueous phase also optionally comprises an emulsifying surfactant.

20. Cosmetic composition according to any one of claims 1 to 19, in which the aqueous phase also optionally comprises a gelling agent in a concentration of from 0.01 to 5% by weight relative to the total weight of the composition.

21. Cosmetic composition according to any one of claims 1 to 20, in which the aqueous phase consists of a physiologically acceptable medium.

22. Cosmetic composition according to any one of claims 1 to 21, which is in the form of a cosmetic, make-up or care composition that may be applied to the skin, including the scalp, the nails, the hair, the eyelashes, the eyebrows, the eyes, mucous membranes and semi-mucous membranes, and any other area of body or facial skin.

23. Composition according to any one of claims 1 to 22, which is chosen from foundations, face powders, eyeshadows, mascaras, products providing coverage or "soft focus"', antisun products and hair products.

24. Use of the polymer as described in claim 1, to eliminate or reduce the tack of a film or deposit obtained from a composition with an optical effect containing the said polymer.

25. Use of the polymer as described according to claim 1, to maintain the staying power of a film or deposit obtained from a composition with an optical effect containing the said polymer.

26. Use according to claim 23 or 24, in which the said film or deposit is exposed to a hot and/or humid atmosphere.

27. Use according to claim 26, in which the relative humidity (RH) of the said atmosphere is from 40 to 95%.

28. Use according to claim 26 or 27, in which the temperature of the said atmosphere is from 25 to 45°C.

29. Cosmetic use of the composition according to any one of claims 1 to 23, to fade out imperfections in the skin relief and/or to conceal the microreliefs, wrinkles, fine lines and/or pores of the skin.

30. Cosmetic use of the composition according to any one of claims 1 to 23, for making up the skin, the eyelashes, the lips and/or the hair.

31. Cosmetic process for treating the skin, which is intended to give it a matt appearance and/or to conceal the defects in the skin relief, **characterized in that** the composition according to any one of claims 1 to 23 is applied to the skin.

## Patentansprüche

1. Kosmetische Zusammensetzung, die eine wässrige Phase aufweist, wobei die wässrige Phase mindestens eine Verbindung mit optischer Wirkung, die unter den Füllstoffen, Pigmenten, Perlglanzstoffen, mattierenden Polymeren und deren Gemischen ausgewählt ist, und ein Polymer enthält, das wasserlösliche Einheiten und Einheiten aufweist, die in Wasser eine untere kritische Entmischungstemperatur LCST aufweisen, wobei die Temperatur der Entmischung durch Erwärmen der LCST-Einheiten in wässriger Lösung für eine Massekonzentration in Wasser von 1 bis 25 % der Einheiten 5 bis 40°C beträgt und wobei das Polymer in Form eines Sequenzpolymers (oder Blockpolymers), das Sequenzen enthält, die aus wasserlöslichen Einheiten bestehen, die mit Sequenzen alternieren, die aus LCST-Einheiten bestehen, oder in Form eines Pfropfpolymers vorliegt, dessen Grundgerüst aus wasserlöslichen Einheiten gebildet wird, wobei das Grundgerüst Pfropfzweige trägt, die aus LCST-Einheiten bestehen; wobei die Polymere partiell vernetzt sein können; und wobei der Masseanteil der LCST-Einheiten des Polymers 5 bis 70 %, bezogen auf das Polymer, beträgt.

2. Kosmetische Zusammensetzung nach Anspruch 1, wobei die Temperatur der Entmischung durch Erwärmen der LCST-Einheiten des Polymers in wässriger Lösung für eine Massekonzentration in Wasser von 1 bis 25 % der Einheiten 10 bis 35 °C beträgt.

3. Kosmetische Zusammensetzung nach einem der vorhergehenden Ansprüche, in der die wasserlöslichen Einheiten ganz oder teilweise durch Polymerisation oder durch Polykondensation erhalten werden können oder ganz oder teilweise aus natürlichen oder modifizierten natürlichen Polymeren bestehen.

4. Kosmetische Zusammensetzung nach Anspruch 3, in der die wasserlöslichen Einheiten ganz oder teilweise durch Polymerisation und insbesondere radikalische Polymerisation mindestens eines Monomers erhältlich sind, das unter den folgenden Monomeren ausgewählt ist:
- (Meth)acrylsäure;
- Vinylmonomeren der folgenden Formel (I): 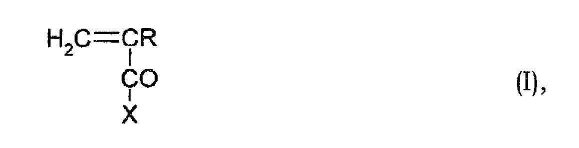 worin bedeuten:
• R ist unter H, -CH₃, -CH₂H₅ oder -C₃H₇ ausgewählt und - X ist ausgewählt unter:
• Alkoxygruppen vom Typ -OR', wobei R' eine geradkettige oder verzweigte, gesättigte oder ungesättigte Kohlenwasserstoffgruppe mit 1 bis 6 Kohlenstoffatomen bedeutet, die gegebenenfalls mit mindestens einem Halogenatom (Iod, Brom, Chlor, Fluor); einer Sulfonsäuregruppe (-SO₃⁻), einer Sulfatgruppe (-SO₄⁻), einer Phosphatgruppe (-PO₄H₂); einer Hydroxygruppe (-OH); einer primären Aminogruppe (-NH₂); einer sekundären Aminogruppe (-NHR₁), einer tertiären Aminogruppe (-NR₁R₂) oder einer quartären Aminogruppe (-N⁺R₁R₂R₃) substituiert ist, wobei R₁, R₂ und R₃ unabhängig voneinander eine geradkettige oder verzweigte, gesättigte oder ungesättigte Kohlenwasserstoffgruppe mit 1 bis 6 Kohlenstoffatomen bedeuten, mit der Maßgabe, dass die Anzahl der Kohlenstoffatome von R' + R₁ + R₂ + R₃ 7 nicht übersteigt; und
• den Gruppen -NH₂, -NHR₄ und -NR₄R₅, wobei R₄ und R₅ unabhängig voneinander geradkettige oder verzweigte, gesättigte oder ungesättigte Kohlenwasserstoffgruppen mit 1 bis 6 Kohlenstoffatomen bedeuten, mit der Maßgabe, dass die Gesamtzahl der Kohlenstoffatome von R₄ + R₅ 7 nicht übersteigt, wobei R₄ und R₅ gegebenenfalls substituiert sind mit einem Halogenatom (Iod, Brom, Chlor, Fluor); einer Hydroxygruppe (-OH); einer Sulfonsäuregruppe (-SO₃⁻) ; einer Sulfatgruppe (-SO₄⁻); einer Phosphatgruppe (-PO₄H₂); einer primären Aminogruppe (-NH₂); einer sekundären Aminogruppe (-NHR₁), einer tertiären Aminogruppe (-NR₁R₂) und/oder einer quartären Aminogruppe (-N⁺R₁R₂R₃), wobei R₁, R₂ und R₃ unabhängig voneinander eine geradkettige oder verzweigte, gesättigte oder ungesättigte Kohlenwasserstoffgruppe mit 1 bis 6 Kohlenstoffatomen bedeuten, mit der Maßgabe, dass die Summe der Kohlenstoffatome von R₄ + R₅ + R₁ + R₂ + R₃ 7 nicht übersteigt;
- Maleinsäureanhydrid;
- Itaconsäure;
- Vinylalkohol der Formel CH₂=CHOH;
- Vinylacetat der Formel CH₂=CH-OCOCH₃;
- N-Vinyllactamen, wie N-Vinylpyrrolidon, N-Vinylcaprolactam und N-Butyrolactam;
- Vinylethern der Formel CH₂=CHOR₆, wobei R₆ eine geradkettige oder verzweige, gesättigte oder ungesättigte Kohlenwasserstoffgruppe mit 1 bis 6 Kohlenstoffatomen ist;
- wasserlöslichen Styrolderivaten, insbesondere Styrolsulfonat;
- Dimethyldiallylammoniumchlorid;
und
- Vinylacetamid.

5. Kosmetische Zusammensetzung nach Anspruch 3, wobei die wasserlöslichen Einheiten des Polymers ganz oder teilweise aus Polykondensaten oder aus natürlichen oder modifizierten natürlichen Polymeren gebildet sind, die unter einer oder mehreren der folgenden Verbindung ausgewählt sind:
- wasserlöslichen Polyurethanen;
- Xanthangummi;
- Alginaten und deren Derivaten, wie Propylanglycolalginat;
- Cellulosederivaten und insbesondere Carboxymethylcellulose, Hydroxypropylcellulose, Hyroxyethylcellulose und quaternisierter Hydroxyethylcellulose;
- Galactomannanen und deren Derivaten, wie Konjac-Gummi, Guargummi, Hydroxypropylguar, mit Natriummethylcarboxylatgruppen modifiziertes Hydroxypropylguargummi und Guarhydroxypropyltrimethylammoniumchlorid; und
- Polyethylenimin.

6. Kosmetische Zusammensetzung nach einem der Ansprüche 4 bis 5, wobei die wasserlöslichen Einheiten des Polymers eine Molmasse von 1 000 bis 5 000 000 g/mol aufweisen, wenn sie das wasserlösliche Grundgerüst eines Propfpolymers bilden, oder eine Molmasse von 500 bis 100 000 g/mol, wenn sie einen Block eines Multiblockpolymers bilden.

7. Kosmetische Zusammensetzung nach einem der Ansprüche 1 bis 6, wobei die LCST-Einheiten des Polymers aus einem oder mehreren Polymeren bestehen, die unter den folgenden Polymeren ausgewählt sind:
- Polyethern, wie Polyethylenoxid (PEO), Polypropylenoxid (PPO) und statistischen Copolymeren von Ethylenoxid (EO) und Propylenoxid (PO);
- Polyvinylmethylethern;
- N-substituierten Polymer- oder Copolymerderivaten von Acrylamid mit LCST-Einheiten; und
- Polyvinylcaprolactam und Copolymeren von Vinylcaprolactam.

8. Kosmetische Zusammensetzung nach einem der Ansprüche 1 bis 7, wobei die LCST-Einheiten des Polymers aus Polypropylenoxid (PPO)ₙ bestehen, wobei n eine ganze Zahl von 10 bis 50 bedeutet, oder statistischen Copolymeren von Ethylenoxid (EO) und Propylenoxid (PO), die durch die folgende Formel dargestellt werden:
(EO)ₘ (PO)ₙ,
wobei m eine ganze Zahl von 1 bis 40 und vorzugsweise 2 bis 20 bedeutet und n eine ganze Zahl von 10 bis 60 und vorzugsweise 20 bis 50 ist.

9. Kosmetische Zusammensetzung nach Anspruch 8, wobei die Molmasse der LCST-Einheiten des Polymers 500 bis 5 300 g/mol und vorzugsweise 1 500 bis 4 000 g/mol beträgt.

10. Kosmetische Zusammensetzung nach Anspruch 9, wobei die LCST-Einheiten des Polymers aus einem N-substituierten Polymer- oder Copolymerderivat von Acrylamid mit LCST-Einheit bestehen.

11. Kosmetische Zusammensetzung nach Anspruch 10, wobei die LCST-Einheiten des Polymers aus einem Polymer gebildet sind, das ausgewählt ist unter Poly-N-Isopropylacrylamid, Poly-N-Ethylacrylamid und den Copolymeren von N-Isopropylacrylamid oder N-Ethylacrylamid und einem Vinylmonomer, das ausgewählt ist unter den Monomeren der in Anspruch 4 angegebenen Formel (I), Maleinsäureanhydrid, Itaconsäure, Vinylpyrrolidon, Styrol und seinen Derivaten, Dimethyldiallylammoniumchlorid, Vinylacetamid, Vinylalkohol, Vinylacetat, Vinylethern und Vinylacetatderivaten.

12. Kosmetische Zusammensetzung nach Anspruch 10 oder 11, wobei die Molmasse der LCST-Einheiten des Polymers 1 000 bis 500 000 g/mol und vorzugsweise 2 000 bis 50 000 g/mol beträgt.

13. Kosmetische Zusammensetzung nach einem der Ansprüche 1 bis 7, wobei die LCST-Einheiten des Polymers aus einem Polyvinylcaprolactam oder einem Copolymer von Vinylcaprolactam und einem Vinylmonomer gebildet sind, das unter den Monomeren der in Anspruch 4 angegebenen Formel (I), Maleinsäureanhydrid, Itaconsäure, Vinylpyrrolidon, Styrol und seinen Derivaten, Dimethyldiallylammoniumchlorid, Vinylacetamid, Vinylalkohol, Vinylacetat, Vinylethern und Vinylacetatderivaten ausgewählt ist.

14. Kosmetische Zusammensetzung nach Anspruch 13, wobei die Molmasse der LCST-Einheiten 1 000 bis 500 000 g/mol und vorzugsweise 2 000 bis 50 000 g/mol beträgt.

15. Kosmetische Zusammensetzung nach einem der Ansprüche 1 bis 14, wobei der Masseanteil der LCST-Einheiten des Polymers 20 bis 65 % und besser 30 bis 60 %, bezogen auf das Polymer, beträgt.

16. Kosmetische Zusammensetzung nach einem der Ansprüche 1 bis 15, wobei die Massekonzentration des Polymers der wässrigen Phase 0,1 bis 20 % und vorzugsweise 0,5 bis 10 % beträgt.

17. Kosmetische Zusammensetzung nach einem der Ansprüche 1 bis 16, bei der es sich um eine Dispersion handelt.

18. Kosmetische Zusammensetzung nach einem der Ansprüche 1 bis 16, die eine Öl-in-Wasser-Emulsion ist und neben der wässrigen Phase eine in der wässrigen Phase dispergierte Ölphase aufweist.

19. Kosmetische Zusammensetzung nach Anspruch 18, wobei die wässrige Phase ferner gegebenenfalls einen emulgierenden grenzflächenaktiven Stoff enthält.

20. Kosmetische Zusammensetzung nach einem der Ansprüche 1 bis 19, wobei die wässrige Phase gegebenenfalls ferner einen Gelbildner in einer Konzentration von 0,01 bis 5 Gew.-%, bezogen auf das Gesamtgewicht der Zusammensetzung, enthält.

21. Kosmetische Zusammensetzung nach einem der Ansprüche 1 bis 20, wobei die wässrige Phase aus einem physiologisch akzeptablen Medium besteht.

22. Kosmetische Zusammensetzung nach einem der Ansprüche 1 bis 21, die in Form einer kosmetischen Zusammensetzung zum Schminken oder für die Pflege vorliegt, die auf die Haut einschließlich der Kopfhaut, die Nägel, die Haare, die Wimpern, die Augenbrauen, die Augen, die Schleimhäute und die Semischleimhäute und alle anderen Hautbereiche des Körpers und des Gesichts aufgetragen werden kann.

23. Zusammensetzung nach einem der Ansprüche 1 bis 22, die unter Make-up, Wangenrouge, Lidschatten, Mascara, Produkten, die abdecken können, oder "Soft Focus"-Produkten, Sonnenschutzprodukten und Produkten für die Haarbehandlung ausgewählt ist.

24. Verwendung des Polymers nach Anspruch 1, um das Oberflächenklebevermögen eines Films oder einer Abscheidung, die aus einer Zusammensetzung mit optischer Wirkung, die das Polymer enthält, hergestellt sind, zu vermindern oder zu beseitigen.

25. Verwendung des Polymers nach Anspruch 1, um die Haftung eines Films oder einer Abscheidung, die aus einer Zusammensetzung mit optischer Wirkung, die das Polymer enthält, hergestellt sind, zu erhalten.

26. Verwendung nach Anspruch 23 oder 24, wobei der Film oder die Abscheidung einer warmen und/oder feuchten Atmosphäre ausgesetzt ist.

27. Verwendung nach Anspruch 26, wobei die relative Feuchte (RH) der Atmosphäre 40 bis 95 % beträgt.

28. Verwendung nach Anspruch 26 oder 27, wobei die Temperatur der Atmosphäre 25 bis 45 °C ist.

29. Kosmetische Verwendung der Zusammensetzung nach einem der Ansprüche 1 bis 23, um Unvollkommenheiten des Hautreliefs zu überdecken und/oder Mikroreliefe, Falten, Fältchen und/ oder Poren der Haut zu verbergen.

30. Kosmetische Verwendung der Zusammensetzung nach einem der Ansprüche 1 bis 23 zum Schminken der Haut, der Wimpern, der Lippen und/oder der Haare.

31. Verfahren zur kosmetischen Behandlung der Haut, das dazu dient, die Haut matt aussehen zu lassen und/oder Mängel des Hautreliefs zu kaschieren, **dadurch gekennzeichnet, dass** auf die Haut die Zusammensetzung nach einem der Ansprüche 1 bis 23 aufgetragen wird.
